# EUROPEAN PATENT SPECIFICATION

(11) **EP 1 466 972 B1**
(45) Date of publication and mention of the grant of the patent: **28.02.2007**
(21) Application number: 02790919.1
(22) Date of filing: 27.12.2002
(51) Int. Cl.: C12N 15/09, C12P 21/02, C12N 15/67

(54) **NUCLEOTIDE SEQUENCES HAVING ACTIVITY OF CONTROLLING TRANSLATION EFFICIENCY AND UTILIZATION THEREOF**
NUKLEOTIDSEQUENZEN MIT AKTIVITÄT ZUR STEUERUNG DER TRANSLATIONSEFFIZIENZ UND DEREN NUTZUNG
SEQUENCES DE NUCLEOTIDES DONT L'ACTIVITE EST DE CONTROLER L'EFFICACITE DE TRADUCTION, ET UTILISATION DE CES SEQUENCES

(30) Priority: 27.12.2001 JP 2001396941
(43) Date of publication of application: 13.10.2004
(73) Proprietor: CellFree Sciences Co., Ltd., Yokohama-shi, Kanagawa 230-0046 (JP)
(72) Inventor: ENDO, Yaeta, Matsuyama-shi, Ehime 791-8016 (JP); SAWASAKI, Tatsuya, Matsuyama-shi, Ehime 790-0811 (JP)
(74) Representative: De Clercq, Ann G. Y.
(86) International application number: PCT/JP2002/013756
(87) International publication number: WO 2003/056009

(56) References cited:
- WO-A1-00/68412
- WO-A1-01/27260
- JP-A- 7 203 984
- DATABASE EMBL [Online] 3 June 2004 (2004-06-03), "Mus musculus molossinus DNA, clone:MSMg01-288E08.T7, genomic survey sequence." XP002331811 retrieved from EBI accession no. EM_PRO:AG418141 Database accession no. AG418141
- DATABASE EMBL [Online] 27 May 2002 (2002-05-27), "Mus musculus BAC clone RP23-459B8 from chromosome 8, complete sequence." XP002331812 retrieved from EBI accession no. EM_PRO:AC122370 Database accession no. AC122370
- DATABASE EMBL [Online] 1 February 2002 (2002-02-01), "Sequence 19 from Patent WO0200932." XP002331813 retrieved from EBI accession no. EM_PRO:AX344568 Database accession no. AX344568
- MADIN K. ET AL.: 'A highly efficient and robust cell-free protein synthesis system prepared from wheat embryos: plants apparently contain a suicide system dirested at ribosomes' PROC. NATL. ACAD. SCI. USA vol. 97, no. 2, January 2000, pages 559 - 564, XP002948543
- OGASAWARA T. ET AL.: 'A new class of enzyme acting on damaged ribosomes: ribosomal RNA apurinic site specific lyase found in wheat germ' EMBO JOURNAL vol. 118, no. 22, 1999, pages 6522 - 6531, XP002966323

## Description

### TECHNICAL FIELD

The present invention relates to a method for screening for a nucleotide sequence having an activity of regulating the translational efficiency of a template in a protein synthesis system, a method for preparing a polynucleotide comprising the nucleotide sequence, use of the polynucleotide, and the like.

### BACKGROUND ART

Intracellular protein synthesis reactions proceed through the steps of first transcribing genetic information from DNA that bears the information into mRNA, and then translating this information of the mRNA in a ribosome so as to synthesize a protein. Methods for performing this intracellular protein synthesis in vitro are actively being developed, wherein components including, for instance, ribosomes and the like, which are intracellular protein translation apparatus, are extracted from an organism, and a template for transcription or translation (hereinafter referred to generally as "template"), nucleic acids and amino acids, which serve as substrates, an energy source, various ions, a buffering solution, and other effective factors are added to this extract to perform the synthesis *in vitro* (hereinafter this series of manipulations may be referred to as a "cell-free protein synthesis system") (JP-6-98790-A, JP-6-225783-A, JP-7-194-A, JP-9-291-A, JP-7-147992-A, etc.).

Cell-free protein synthesis systems have capabilities comparable to those of living cells in terms of the rate of peptide synthesis and the accuracy of the translational reaction, and have the advantages of not requiring complex chemical reaction processes and cumbersome cell culture processes. In addition, recently, in order to further increase translational efficiency, such developments are also under way as that of inactivating a group of nucleases, translation inhibitory protein factors, and proteolytic enzymes, etc., that contaminate the extract obtained from cells or tissues used in a conventional cell-free protein synthesis system (JP-2000-236896-A), or that of preventing the contamination thereof (JP-2000-236896-A).

Meanwhile, in terms of improvement of protein synthesis efficiency, use of a nucleotide sequence that improves the transla6onal efficiency *per se* is also known. Such a translation promoting sequence includes the 5' cap structure (Shatkin, Cell, 9, pp. 645- (1976)). Kozak sequence (Kozak, *Nucleic Acid. Res.,* 12, pp. 857- (1984)) and the like in eukaryotes. The Shine-Dalgamo sequence and the like are known in prokaryotes. Moreover, translation promoting activity has been found to be present in the 5'-untranslated leader sequences of RNA viruses (JP-2814433-B), and a method has been developed for efficient protein synthesis using these sequences (JP-10-146197-A).

However, since these translation promoting sequences have specificity to the RNA polymerase that carries out the transcription, they are not necessarily suited to exploitation in protein synthesis. Furthermore, no examples are known of artificially randomized non-natural nucleotide sequences having translational efficiency regulatory activity.

Therefore, an object of the present invention is to provide a novel nucleotide sequence having the activity of regulating the translational efficiency of a template in a cell-free protein synthesis system, and a method for obtaining a polynucleotide containing the nucleotide sequence, and also to provide a more effective protein synthesis method or the like that uses as a template a nucleic acid molecule containing the polynucleotide.

### DISCLOSURE OF THE INVENTION

As a result of extensive research directed at solving the aforementioned problems, the present inventors discovered that, after carrying out a cell-free protein synthesis with wheat germ extract, using a template for synthesizing a luciferase protein containing a polynucleotide which has a random sequence of 22 mer and 57 mer in the 5' non-translated region, when the polyribosomal fraction from the reaction solution was recovered by centrifugation over a sucrose density gradient, a nucleotide sequence analysis of the random sequence contained in the template RNA of the fraction was performed, and protein synthesis was performed using a template that contained a polynucleotide comprising the nucleotide sequence, translational efficiency was increased. The present invention was completed based on these observations.

That is to say, the present invention provides:
1. a polynucleotide having translation enhancement activity, comprising the nucleotide sequence set forth in SEQ ID NO: 11;
2. the polynucleotide recited above in 1, which comprises the nucleotide sequence set forth in SEQ ID NO: 136;
3. a nucleic acid molecule appropriate for translation of a polynucleotide coding for a target polypeptide, comprising the polynucleotide recited above in any of 1 or 2 upstream of the said coding polynucleotide;
4. the nucleic acid molecule recited above in 3, further comprising a promoter sequence upstream of the polynucleotide recited above in any of 1 or 2;
5. the nucleic acid molecule recited above in 4, further comprising a 3' non-translated region downstream of the said coding polynucleotide;
6. a nucleic acid molecule having translation enhancement activity obtainable by transcribing the nucleic acid molecule recited above in any of 4 or 5 from the said promoter sequence;
7. a vector comprising the polynucleotide recited above in any of 1 or 2 and/or the nucleic acid molecule recited above in any of 3 to 5;
8. a protein synthesis method characterised by the use of the nucleic acid molecule recited above in any of 3 to 5 or the vector recited above in 7 as a template for transcription and/or translation;
9. a protein synthesis method characterised by the use of the nucleic acid molecule recited above in 6 as a template for translation.

### BRIEF DESCRIPTION OF THE DRAWINGS

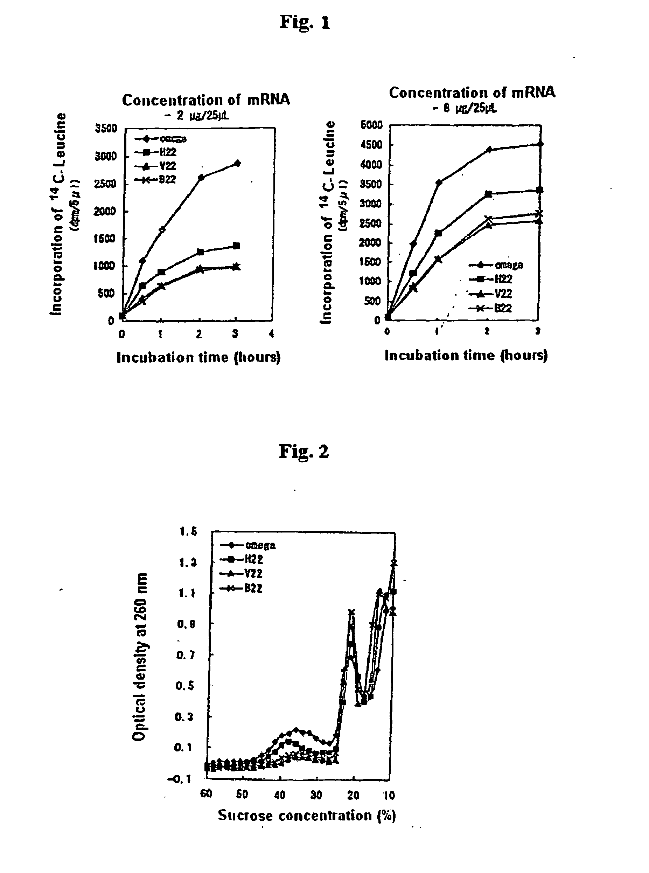

Fig. 1 shows the template activity of a translation template that contains a 22-mer random sequence having an H, V or B base composition. The vertical axis indicates the incorporation of ¹⁴C-Leu (dpm/5µl), and the horizontal axis indicates the incubation time (hours).
Fig. 2 shows the optical density at 260nm of each fraction from a protein synthesis reaction solution, which was fractionated by centrifugation over a 10-60% sucrose density gradient. The sharp peak indicates a fraction containing the 80S ribosome, and the fractions containing the broad peak at a higher concentration indicate fractions that contain polyribosomes.

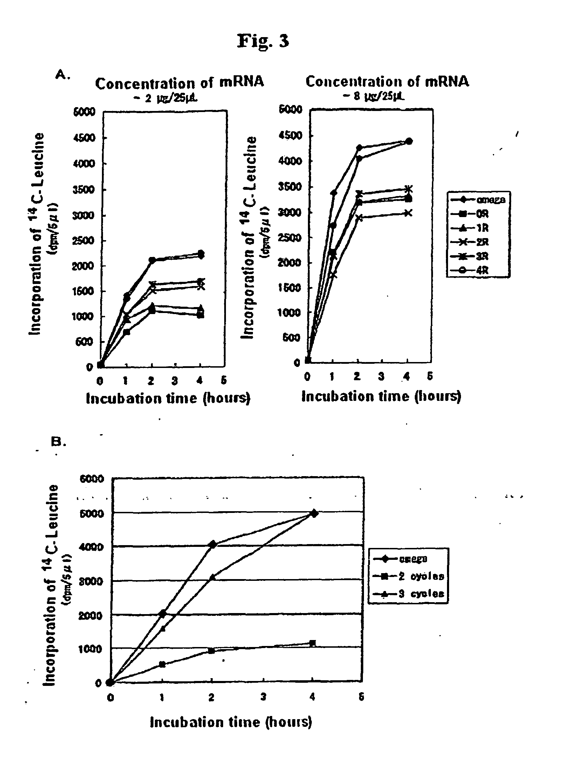

Fig. 3 indicates the template activity of a translation template containing a random sequence of 22 mer (A) and 57 mer (B) with the H base composition, after each cycle. The vertical axis indicates the incorporation of ¹⁴C-Leu (dpm/5µl), and the horizontal axis indicates the incubation time (hours).

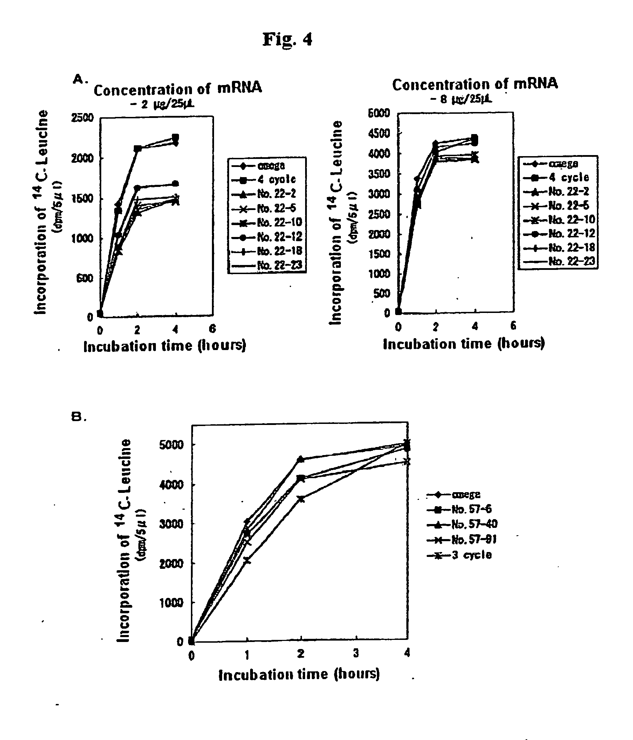

Fig. 4 shows the template activity of a translation template that contains a variety of 22-mer sequences of the H base composition screened after 4 cycles (A) and a variety of 57-mer sequences of the H base composition screened after 3 cycles (B). The vertical axis indicates the incorporation of ¹⁴C-Leu (dpm/5µl), and the horizontal axis indicates the incubation time (hours).

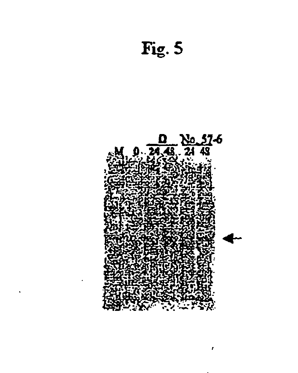

Fig. 5 shows the template activity of a translation template containing a 57-mer sequence of the H base composition (Nd.57-6), when the protein synthesis reaction was carried out in a dialysis system. After fixed reaction times (24 and 48 hours) the protein synthesis reaction solution was collected and electrophoresed over SDS-polyacrylamide gel, and then the proteins were stained with CBB. The arrow indicates the band of the target protein (GFP). M indicates a molecular weight marker, while Ω indicates the protein synthesis reaction solution wherein a translation template containing the Ω sequence was used.

### DESCRIPTION OF THE PREFERRED EMBODIEMNT

As described above, the present invention relates to a method for screening for a nucleotide sequence having the activity of regulating the translation efficiency of a template in a protein synthesis system, characterized by (a) supplying one or more types of template comprising any nucleotide sequence to a protein synthesis reaction system, (b) after the reaction, recovering a polyribosomal fraction from the reaction solution, and (c) analyzing the nucleotide sequence present in the template contained in the polyribosomal fraction. The present invention further relates to a method for preparing a polynucleotide having the activity of regulating translational efficiency characterized by collecting, at the above step (c), a polynucleotide containing a nucleotide sequence from the templates contained in the polyribosomal fraction; and relates to use of the polynucleotide. Hereinafter, the present invention will be described in more detail.

### (1) Fabrication of a template containing any nucleotide sequence

The nucleotide sequence to be used in the present invention can be any sequence, so long as it has the activity of regulating translational efficiency; examples include a sequence contained in the 5' non-translated region of the known gene and the like; preferably, this is an artificially synthesized random sequence without a start codon, having a length of 3 mer to 200 mer, and more preferably of 10 mer to 200 mer.

Examples of a method for fabricating a polynucleotide group having the sequence in question (hereinafter, this may be referred to as a "candidate polynucleotide") include, in the case of a random sequence, a method wherein a chemical synthesis is carried out by replacing a column used in a conventional oligonucleotide synthesis method with a column having a mixture of nucleic acids containing four different kinds of bases, or the like. Herein, to obtain random sequences that do not contain a start codon, such methods are preferably used as those wherein synthesis is by way of a mixture of nucleic acids lacking any one or more of A, T or G from the aforementioned four kinds of bases, or a mixture of nucleic acids wherein any one thereof is replaced with inosine. In addition, if a random sequence is to be used, it is preferred that a consensus sequence be added at the 5' end thereof in order to analyze the nucleotide sequence of the polynucleotide, or to amplify it by polymerase chain reaction (PCR). There is no particular restriction on the consensus sequence as long as the sequence does not have a start codon and contain sequence which can be annealed by a PCR primer. A preferable chain length is 3 mer to 50 mer.

The template (transcription template) containing the candidate polynucleotide is created by linking this polynucleotide so as to be sandwiched between a suitable promoter sequence and a polynucleotide which contains a start codon and a nucleotide sequence that codes for a polypeptide (herein, this may be referred to as "coding polynucleotide"). The polypeptide coded by the coding polynucleotide may be any polypeptide as long as it can be synthesized in a protein synthesis system. However, since the amount of polypeptide synthesized is an indicator of the strength of the translation efficiency resulting from the candidate polynucleotide, a polypeptide that emits an easily observable signal such as fluorescence or chemiluminescence by itself or as a result of a reaction in which it is involved is preferred. Furthermore, a polypeptide wherein the strength of its signal correlates to the amount of protein is preferred. Examples of such polypeptides include luciferase.

It is preferable that the coding polynucleotide contain not only the coding region of the above-mentioned polypeptide, but also the 3' non-translated region that includes the transcription termination region thereof, etc. It is preferable that the 3' non-translated region be on the order of approximately 1.0 to 3.0 kilo bases downstream from the stop codon. These 3' non-translated regions need not be derived from the gene coding for the polypeptide. In addition, a promoter specific to the RNA polymerase that is to be used in later translation can be used for the promoter. Specifically, the SP6 promoter, the T7 promoter and the like may be cited as examples thereof.

A conventional method well known *per se* can be used as the method for linking a promoter with a coding polynucleotide and a candidate polynucleotide. Specifically, a method can be used to link a promoter and a candidate polynucleotide, wherein the synthesis of the 5'-side promoter sequence continues when the candidate polynucleotide is chemically synthesized. In addition, methods used for linking with a coding polynucleotide include those wherein the coding polynucleotide is used as a template, a candidate polynucleotide that has been synthesized to be linked the promoter sequence is used as the sense primer, the polynucleotide comprising the 3' end sequence of the 3' non-translated region is used as the antisense primer, and PCR is carried out to link each of these.

In addition, a known nucleotide sequence having the activity of regulating transcriptional and translational efficiencies can be inserted. Examples of such a sequence and such an insertion site include, in eukaryotes, insertion of the 5' cap structure (Shatkin, *Cell,* 9, pp. 645- (1976)) at the 5' end of the translation template, insertion of the Kozak sequence (Kozak, *Nucleic Acid. Res.,* 12, pp. 857- (1984)) between the candidate polynucleotide of the present invention and the coding polynucleotide, or the like. Furthermore, in prokaryotes, examples include insertion of the Shine-Dalgamo sequence between the candidate polynucleotide of the present invention and the coding polynucleotide, or the like.

### (2) Protein synthesis reaction using the template

The aforementioned template containing the candidate polynucleotide of the present invention (transcription template) may, if necessary, be separately subjected to a transcription reaction, in order to convert this into a translation template (RNA), and then supplied to the protein synthesis reaction, or if the protein synthesis system can perform the transcription reaction at the same time, the template can be supplied to the reaction system as it is. The protein synthesis system to be used may be any protein synthesis system as long as it has the capability of translating the translation template and generating a protein, and specific examples include a living cell and a cell-free protein synthesis system. Known systems such as cell extracts, or the like, from *Escherichia coli,* plant seed germ, rabbit reticulocyte and the like may be used as the cell-free protein synthesis system. Commercially available systems may be used, or these can be prepared by methods well known *per se;* specifically, *Escherichia coli* extracts can be prepared according to the method described in Pratt, J. M., *et al., Transcription and Translation,* 179-209, Hames, B. D. & Higgins, S. J. eds.), IRL Press, Oxford (1984), or the like.

In terms of commercially available cell-free protein synthesis systems and cell extracts, those derived from *Escherichia coli* include the E. coli. S30 extract system (Promega), the RTS 500 Rapid Translation System (Roche) and the like, while those derived from rabbit reticulocyte include the Rabbit Reticulocyte Lysate Sytem (Promega) and the like. Furthermore, those derived from wheat germ include PROTEIOS^{™} (TOYOBO) and the like. Among these, the use of a plant seed germ extract system is preferred, and as plant seeds, those from plants of the *Poaceae* family such as wheat, barley, rice and com, and from spinach or the like are preferred. In the cell-free synthesis system of the present invention, since a protein synthesis system with high polyribosome formation activity is preferred, those using a wheat germ extract are most preferred.

As a method for preparing a wheat germ extract, methods described, for instance, in Johnston, F. B. *et al., Nature,* 179, pp. 160-161 (1957), or Erickson, A. H. *et al.,* (1996) *Meth. In Enzymol.,* 96, pp. 38-50, and the like can be used. Furthermore, a treatment to remove the endosperm contained in the extract, which contains translation inhibitory factors, such as tritin, thionine, nuclease and the like (JP-2000-236896-A), or a treatment to inhibit the activation of translation inhibitory factors (JP-7-203984-A) may preferably be performed. The cell extract obtained in this manner can be used in a protein synthesis system in the same way as in a conventional method.

A composition of the synthesis reaction solution used in the protein synthesis system of the present invention includes the aforementioned cell extract, a translation template containing the candidate polynucleotide, amino acids serving as substrates, an energy source, various ions, a buffering solution, an ATP regeneration system, a nuclease inhibitor, tRNAs, a reducing agent, polyethylene glycol, 3', 5'-cAMP, folate, an antibacterial agent and the like. In addition, if the transcription and the translation reactions are to be carried out together using DNA as a template, the reaction solution may further contain a substrate for RNA synthesis, an RNA polymerase and the like. These are suitably selected and prepared according to the type of the target protein and the type of protein synthesis system to be used. The amino adds serving as the substrates are the 20 types of amino adds constituting the proteins. In addition, examples of energy sources include ATP and GTP. Examples of various ions include acetates such as potassium acetate, magnesium acetate and ammonium acetate, glutamic acid salt and the like. Hepes-KOH, Tris-acetate and the like can be used as a buffer solution. In addition, examples of ATP regeneration systems include the combination of phosphoenol pyruvate and pyruvic acid kinase, and the combination of creatine phosphate and creatine kinase. Examples of nuclease inhibitors include ribonuclease inhibitors, nuclease inhibitors and the like. Among these, as a specific example of ribonuclease inhibitor, human placenta-derived RNase inhibitor (TOYOBO) and the like can be used. tRNAs may be obtained by the method described in Monitor, R., *et al., Biochim. Biophys. Acta.,* 43, 1 (1960) etc., or those that are commercially available can be used. Dithiothreitol or the like can be cited as a reducing agent. Examples of antibacterial agents include sodium azide, ampicillin and the like. In addition, an RNA polymerase that is suitable for the promoter contained in the template is used; specifically, for instance, SP6 RNA polymerase, T7 RNA polymerase and the like can be used. The amount of these compounds to be added is selected suitably to prepare the synthesis reaction solution.

The protein synthesis solution obtained in this way is introduced into a correspondingly selected system or apparatus that is well known *per se*, so as to carry out protein synthesis. Systems and apparatus for protein synthesis include the batch method (Pratt, J. M. *et al., Transcription and Translation,* 179-209, Hames, B. D. & Higgins, S. J., eds.), IRL Press, Oxford (1984)), or the continuous cell-free protein synthesis system which continuously supply the amino acids, the energy source and the like to the reaction system (Spirin, A. S., *et al., Science,* 242, 1162-1164 (1988)), the dialysis method (Kikawa *et al., 21st Meeting of the Molecular Biology Society of Japan,* WID6), or the bi-layer method (Specification, Japanese Patent Application 2000-259186). Furthermore, a method wherein the template RNA, the amino acids, the energy source and the like are supplied to the reaction system at the necessary time, and the synthesized products and degraded products are removed at the necessary time (JP-2000-333673-A; hereinafter this may be referred to as the "discontinuous gel filtration method"), or a method wherein the synthesis reaction chamber is prepared with a support that can function as molecular sieve, the synthesized material or the like is developed with the support as the mobile phase, the synthesis reaction is performed during the development, and the synthesized protein may be recovered as the result (JP-2000-316595-A), and the like, can be used. Since the protein synthesis reaction used in the screening the nucleotide sequence of the present invention, having the activity of regulating translational efficiency, has the objective of forming polyribosomes, the batch method is considered to be sufficient.

When protein synthesis is carried out by the batch method, the synthesis can, for instance, be achieved by adding a template to the above-mentioned synthesis reaction solution from which the template has been omitted, and incubating the solution. When wheat germ extract is used, incubation is carried out at 10°C to 40°C, preferably at 18°C to 30°C, and more preferably at 20°C to 26°C. If the reaction time is long enough to generate polyribosomes only from templates that have a high polyribosome formation capability, it is possible to screen for nucleotide sequences having translation enhancement activity. Specifically, a range of 5 minutes to 2 hours can be given as preferable reaction times in screening for a nucleotide sequence having translation enhancement activity, and on the order of 30 minutes is the most preferable reaction time. The reaction time can be controlled by arresting the reaction by adding a protein synthesis inhibitory enzyme, but the present invention can also be carried out without arresting the reaction. The protein synthesis inhibitory enzyme may be any inhibitor as long as it is not an inhibitor of the initiation of the translation reaction. Specifically, for example cycloheximide, ribotoxin and the like may be cited. Specifically, a-sarcin (Endo, Y., *et al., J. Biol. Chem.,* 258, pp. 2662-2667 (1983)), ribosome inactivating protein (Endo, Y., *et al., J. Biol. Chem.,* 262, pp. 8128-8130 (1987)) and the like can be cited as ribotoxins. The amount of these inhibitors that is added can be suitably selected according to the protein synthesis system, but when cycloheximide is to be added to a protein synthesis system that uses a wheat germ extract, a final concentration of on the order of 0.5 to 10 µM is preferred.

When protein synthesis is carried out by dialysis, the protein synthesis is carried out using an apparatus that is divided by a dialysis membrane, which allows movement of the external dialysis solution and substances, with the synthesis reaction solution to which the template has been added as the internal dialysis solution. As a specific example, the template is added to the reaction solution, and after pre-incubating for an adequate time, the reaction solution is placed in a suitable dialysis container to be used as the internal reaction solution. Examples of dialysis containers include containers with a dialysis membrane at the bottom (Daiichi Kagaku: Dialysis Cup 12,000 or the like), or a dialysis tube (Sanko Junyaku: 12,000 or the like). The dialysis membrane used should have a molecular weight cutoff of 10,000 Dalton or more, those with a molecular weight cutoff on the order of 12,000 Dalton being preferred. The aforementioned synthesis reaction solution from which the template has been omitted is used as the external dialysis solution. The reaction temperature and time are selected suitably according to the protein synthesis system to be used.

When the protein synthesis is carried out using the bi-layer method, the synthesis reaction solution to which the template has been added is placed in a suitable container, the external dialysis solution described above in the dialysis method is overlaid on top of the solution so as not to disturb the interface, and protein synthesis is performed. As a specific example, the template is added to the synthesis reaction solution, which is placed in a suitable container to be used as a reaction phase. Examples of the container include a microtiter plate or the like. The external dialysis solution described above in the dialysis method (supply phase) is overlaid on the top layer of this reaction phase so as not to disturb the interface, and the reaction is performed. The reaction temperature and time are selected suitably according to the protein synthesis system to be used. In addition, the interface between the two phases does not have to be formed by superposition in a horizontal plane; a horizontal plane can also be formed by centrifuging the mixture that contains both phases. When the diameter of the circular interface between the two phases is 7 mm, a volume ratio of the reaction phase and the supply phase of 1:4 to 1:8 is adequate, and 1:5 is optimal. The rate of exchange of substances due to diffusion increases with the area of the interface formed by the two phases being larger, increasing the protein synthesis efficiency. Therefore, the volume ratio of the two phases changes according to the area of the interface between the two phases. The synthesis reaction should be carried out under static conditions, and the reaction temperature and time are suitably selected for the protein synthesis system to be used. Furthermore, this can be performed at 30 to 37°C when using an *Escherichia coli* extract.

When the protein synthesis is carried out using the discontinuous gel filtration method, the synthesis reaction is carried out by way of the synthesis reaction solution to which a template has been added. At the point of time when the synthesis reaction stops, the template RNA, the amino acids, the energy source and the like are supplied, the products of synthesis or degradation are evacuated, and protein synthesis is performed. As a specific example, a template is added to the synthesis reaction solution, and this is placed in a suitable container so as to carry out the reaction. Examples of the container include a microplate or the like. In this reaction, for instance if the reaction solution contains 48% in volume of wheat germ extract, the synthesis reaction stops completely in one hour. This can be verified by measuring the incorporation of amino acids into the protein or by an analysis of polyribosomes by centrifugation over a sucrose density gradient *(Proc. Natl. Acad Sci. USA,* 97, pp. 559-564 (2000)). The reaction solution in which the synthesis reaction has stopped is passed through a gel filtration column pre-equilibrated with a supply solution which has the same composition as the external dialysis solution described in the dialysis method. The synthesis reaction is resumed by re-incubating the filtered solution at an adequate reaction temperature, and the protein synthesis proceeds over several hours. Thereafter, this reaction and gel filtration manipulation are repeated. The reaction temperature and time are suitably selected for the protein synthesis system to be used.

### (3) Obtaining polyribosomal fractions

The protein synthesis reaction solution using the template containing the candidate polynucleotide of the present invention is fractionated after the reaction, to separate a polyribosomal fraction. Examples of the fractionation method include centrifugation, chromatography, and filtration through a filter. From among these, the centrifugation method is most preferably employed. Examples of the centrifugation method include density gradient centrifugation, equilibrium density gradient centrifugation, and conventional fractionation centrifugation. From among these, the density gradient centrifugation method is most preferably employed.

Density gradient centrifugation is a method wherein a sample solution is overlaid on top of a pre-made density gradient and then centrifuged, and can be performed according to conventional methods well known *per se.* Instruments used to form the density gradient may be commercially available or any apparatus combined according to methods well known *per se,* as long as a stable density gradient can be formed. Furthermore, this can also be formed by overlaying solutions with different concentrations. Sucrose, glycerol, heavy water (D₂O), inorganic salt solutions and the like are used as solvents for forming a density gradient. From among these the use of a sucrose solution is preferred:

The polyribosome fractionation method will be described in detail, using sucrose density gradient centrifugation as an example. The method described in *Proc. Natl. Acad Sci. USA.,* 97, pp. 559-564 (2000) or the like can be used to partition a protein synthesis reaction solution by centrifugation over a sucrose density gradient. Specifically, there are no particular limits on the sucrose concentration gradient so long as it is in a range of concentration that allows polyribosomes to be isolated from the aforementioned protein synthesis reaction solution. Generally, a concentration gradient with a lower limit in the range from 5% to 30% and an upper limit in the range from 30% to the saturation concentration is used. Among these, gradients with concentrations between a lower limit of 10% and an upper limit of 60% are most preferably used. In addition, any buffer solution that can stably maintain the complex of the polyribosome and the translation template may be used to dissolve the sucrose, and specifically, those containing Tris-HCl, potassium chloride, magnesium chloride, cycloheximide and the like may be cited as examples.

A density gradient using the sucrose solution as described above is prepared in an appropriate centrifuge tube or the like, and the protein synthesis solution, which is diluted as necessary with a suitable buffer solution after the reaction is terminated, is overlaid on top of this. It is preferable that the same buffer solution that is used to dissolve the sucrose be used for the suitable buffer solution. Furthermore, there are no particular limits on the degree of the dilution, as long as there is no co-precipitation, but a dilution of on the order of 1 to 100 fold is preferred. The diluted protein synthesis reaction solution can be overlaid in an amount of approximately 1/100 to 100 times the amount of the sucrose solution, and overlaying on the order of 50 times this amount is preferred. This is centrifuged to the extent that polyribosomes can be isolated. Specific examples of the centrifugation conditions include on the order of 30 minutes to 3 hours at 4°C and 80,000 to 400,000 xg. Following centrifugation, the solution is fractionated into appropriate amounts, and the fractions containing the polyribosomes are identified by, for example, measuring the quantity of nucleic acid in each fraction. As a specific example, when density gradient centrifugation is performed with 5 ml sucrose solution and 100 µl protein synthesis reaction solution, OD₂₆₀ is measured for each fraction, with each fraction being 100 to 200 µl. If, for instance, a eukaryote-derived protein synthesis system is used, a peak indicative of the 80S ribosome is present in these measurement values, and fractions such as those centered on a fraction on the higher molecular weight side, for which the measurement values show a peak, are collected as polyribosomal fractions. Examples of such polyribosomal fractions include fractions with sucrose concentrations of 25% to 45%, described in Example 2.

### (4) Screening for a nucleotide sequence having an activity of regulating translation efficiency, and obtaining a polynucleotide comprising the base sequence

A nucleotide sequence that has the activity of regulating translation efficiency can be screened for by obtaining, from the polyribosomal fractions obtained, the RNA (translation template) bound to the polyribosome, reverse-transcribing the RNA to obtain the cDNA, and further analyzing the base sequence of the candidate polynucleotide portion contained in the cDNA. In addition, the aforementioned cDNA contains a polynucleotide comprising the nucleotide sequence that has the activity of regulating translation efficiency. Thus it is, for example, possible to obtain a polynucleotide comprising the nucleotide sequence that has the activity of regulating the translation efficiency by amplifying the sequence portion by PCR or the like.

A method well known *per se* can be used as the method for obtaining the RNA bound to the polyribosome from the polyribosomal fractions. Specifically, the acid guanidium thiocyanate-phenol-chloroform (AGPC) method (Chomczynski P., *et al., Anal. Biochem.,* 162, pp. 156-159 (1987)) is preferably used. Since there is a possibility that the DNA introduced into the protein synthesis system as a template is contained in the solution containing the RNA that was obtained, treatment by a DNA degradation enzyme such as DNase I is preferable.

After purification by a method known in the art, such as phenol/chloroform extraction and ethanol precipitation, the RNA solution that has been obtained can be supplied to a reverse transcription reaction. A commonly used known method can be employed for the reverse transcription reaction; however, considering the cDNA generation efficiency and the like, the use of AMV reverse transcriptase is preferred. In addition, a commercially available kit such as the RNA LA PCR Kit (AMV) ver.1.1 (TAKARA) can also be used.

The cDNA created through the reverse transcription reaction, in itself, contains a polynucleotide that has the activity of regulating the translation efficiency, which can be cloned or amplified. When cloning, the cDNA created above can be inserted into an appropriate vector and cloned. Alternatively, if a consensus sequence has been added when creating a template that contains the candidate polynucleotide, as described above in (1), after amplification by PCR in which this consensus sequence is used as a sense primer and a sequence complementary to the nucleotide sequence at the 5' end of the coding polynucleotide is used as an antisense primer, the cDNA can be inserted into an suitable vector and cloned. By using the polynucleotide cloned in this way as the candidate polynucleotide mentioned above in (1) so as to fabricate a template in the same way, and performing protein synthesis using this template, the translation efficiency regulatory activity of the polynucleotide can be determined. Specifically, as methods for quantifying the amount of synthesized protein, for instance, measurement of amino acids incorporated into the protein, separation by SDS-polyacrylamide electrophoresis and staining with Coomassie brilliant blue (CBB), and autoradiography (Endo, Y., *et al., J. Biotech.,* 25, pp. 221-230 (1992); *Proc. Natl. Acad. Sci. USA,* 97, pp. 559-564 (2000)) can be used. If a template coding for a luminescent enzymatic protein such as luciferase is used as the template of the present invention, a method that measures the intensity of the light emission that is generated by the reaction catalyzed by the protein is preferably used. In addition, by analyzing the nucleotide sequences of the cDNA by a conventional method, the nucleotide sequences having the activity of regulating the translation efficiency can be determined.

### (5) Screening for a nucleotide sequence with still higher translation efficiency regulatory activity and method for obtaining a polynucleotide comprising the nucleotide sequence

By using the cDNA obtained by the method described above in (4) as the candidate polynucleotide mentioned above in (1) and creating a template in the same manner, and then repeating the methods described above in (1) to (4), a polynucleotide with a still higher translation efficiency regulatory activity can be obtained, and the nucleotide sequence thereof can be determined. Furthermore, it is also possible to obtain a polynucleotide with a still higher translation efficiency regulatory activity, and to determine the nucleotide sequence thereof, by introducing a mutation into the cDNA obtained in (4) as described above by a commonly used method known *per se*, and repeating the methods as described above in (1) to (4) using this cDNA with a mutant. Specifically, examples of methods for introducing a mutation into the nucleotide sequence include the error-prone PCR method, and the point mutagenesis method.

Among polynucleotides having translation efficiency regulatory activity obtained in this manner, those comprising the sequences set forth in SEQ ID NO: 11-135 or the like may be given as examples of polynucleotides having translation enhancement activity. These polynucleotides comprise nucleotide sequences that are artificially randomized, and do not contain naturally existing known nucleotide sequences. So long as the polypeptides of the present invention comprise an artificial random nucleotide sequence of 3 to 200 mer in length and have an activity regulating translation efficiency, methods of screening for and obtaining the same are not limited to those described above.

### (6) Protein synthesis using a template containing a polynucleotide having translation enhancement activity

A template that is appropriate for translation can be fabricated by linking a polynucleotide of the present invention, which possesses translation enhancement activity, so that this is sandwiched between a promoter sequence and a coding polynucleotide that codes for the target polypeptide (Sawasaki, T. et al., PNAS, 99 (23), pp. 14652-7 (2002)). It is preferable that the coding polynucleotide contain not only the coding region of the polypeptide, but also the 3' non-translated region containing the transcription termination region thereof and the like. The 3' non-translated region is preferably approximately 0.1 to 3.0 kilobases downstream from the stop codon. In addition, a promoter specific to the RNA polymerase that is used in subsequent transcription can be used. Specifically, the SP6 promoter, the T7 promoter and the like can be given as examples.

The method as described above in (1), the overlap PCR method, or the like, can be used as the method for linking the promoter and the coding polynucleotide to the polynucleotide of the present invention, which possesses translation enhancement activity. The template obtained in this way is supplied to the protein synthesis system in the same way as in the method described above in (2), allowing the target polypeptide to be synthesized. The polypeptide obtained in this way can be identified by a known method *per se.* Specifically, for example, measurement of amino acids incorporated into proteins, separation by SDS-polyacrylamide gel electrophoresis and staining with Coomassie brilliant blue (CBB), autoradiography (Endo, Y., *et al., J. Biotech.,* 25, pp. 221-230 (1992); *Proc. Natl. Acad. Sci. USA,* 97, pp: 559-564 (2000)) and the like, can be used.

In addition, since the reaction solution obtained in this way contains the target protein at a high concentration, the target polypeptide can be obtained by a method known *per se* for isolating and purifying from the reaction solution, such as dialysis, ion exchange chromatography, affinity chromatography or gel filtration.

### (7) Vector containing the polynucleotide having translation enhancement activity

The polynucleotide of the present invention that possesses translation enhancement activity can be inserted into an appropriate vector to fabricate a template for protein synthesis. Examples of vectors to be used include suitable cloning vectors, vectors for use in protein synthesis comprising the T7 promoter or the SP6 promoter and a transcription termination region, and the like.

The present invention will be described in more detail hereinafter by way of examples. The scope of the present invention is not, however, limited to these examples.

### Example 1: Examination of the base composition of candidate polynucleotides (random sequence) containg the nucleotide sequence having translation enhancement activity

### (1) Creation of an mRNA having a candidate polynucleotide (random sequence) with a composition having 3 types of bases

PCR was performed, using as a template a plasmid into which a luciferase gene DNA (pSP-luc+: Promega, catalog NO: E1781) had been inserted, and using a sense primer (SEQ ID NO: 1, 2 or 3) comprising a sequence having a 22-mer random region of a mixed base composition B (T, C and G without A), V (A, C and G without T) or H (A, T and C without G), respectively, the 5' end sequence of the luciferase coding region on the 3' side thereof, a consensus sequence comprising 12 nucleotides on the 5' side of the mixed base composition region, and further an SP6 promoter linked on the 5' side thereof, and also using an antisense primer (SEQ ID NO: 4) containing a sequence that is 3' downstream by 1652 bases from the stop codon of the luciferase gene DNA. The DNA fragment of approximately 3400 bp that was obtained was purified by ethanol precipitation, and was used as a template for transcription using SP6 RNA Polymerase (Promega). After phenol/chloroform extraction and ethanol precipitation of the RNA obtained, this was purified with a Nick Column (Amersham Pharmacia Biotech). This was used as a translation template in the following experiment.

### (2) Preparation of solution containing wheat germ extract

Hokkaido Chihoku wheat grain (not disinfected) was added to a mill (Fritsch: Rotor Speed Mill Pulverisette 14) at a rate of 100g per minute, and the grain was moderately ground at a rotation speed of 8,000 rpm. After recovering a fraction containing germinatable germs with a sieve (mesh size 0.7 to 1.00 mm), the surfacing fraction containing the germinatable germs was recovered by flotation using a mixture of carbon tetrachloride and cyclohexane (volume ratio = carbon tetrachloride : cyclohexane = 2.4 : 1), the organic solvent was eliminated by desiccation at room temperature, and then impurities such as husk were eliminated by air-blowing at room temperature to obtain a crude germ fraction. Wheat germs were judged by eye and selected from this crude germ fraction using a bamboo skewer.

The wheat germ fraction obtained was suspended in distilled water at 4°C, and washed using an ultrasonic washing apparatus until the washing solution was no longer clouded. This was then suspended in a 0.5 % (volume) Nonidet (Nacalai Tectonics) P40 solution and washed using an ultrasonic washing apparatus until the washing solution was no longer clouded, so as to obtain the wheat germs.

A solution containing wheat germ extract was prepared according to a method known in the art (Erickson, A. H., *et al.,* (1996) *Meth. in Enzymol.,* 96, pp. 38-50). The following operations were performed at 4°C. First, wheat germs that were frozen with liquid nitrogen were finely ground in a mortar. An extraction solvent from the method of Patterson *et al.,* which was partly modified (80 mM HEPES-KOH (pH 7.6), 200 mM potassium acetate, 2 mM magnesium acetate, 4 mM calcium chloride, 0.6 mM each of the 20 types of L-type amino acids, and 8 mM dithiothreitol at final concentrations), was added at 1 ml per gram of powder produced, and carefully agitated so as not to generate foam. The supernatant obtained by centrifugation at 30,000xg for 15 minutes was recovered as the embryonic extract and subjected to gel filtration over a Sephadex G-25 column (Amersham Pharmacia Biotech) that was pre-equilibrated with a solution (40 mM HEPES-KOH (pH 7.6), 100 mM potassium acetate, 5 mM magnesium acetate, 0.3 mM each of the 20 types of L-type amino acids, and 4 mM dithiothreitol, at final concentrations). The concentration of the solution containing wheat germ extract obtained in this way was adjusted so as to have an optical density (O.D.) at 260 nm (A260) of 170 to 250 (A260/A280 = 1.5).

### (3) Protein synthesis with a cell-free protein synthesis system (batch method) using wheat germ extract

A volume of 25 µl of a reaction solution for protein synthesis (29 mM HEPES-KOH (pH 7.8), 95 mM potassium acetate, 2.7 mM magnesium acetate, 0.4 mM spermidine (Nacalai Techtonics), 0.23 mM each of the 20 types of L-type amino acids, 2.9 mM dithiothreitol, 1.2 mM ATP (Wako Pure Chemical), 0.25 mM GTP (Wako Pure Chemical), 15 mM creatine phosphate (Wako Pure Chemical), 0.9 U/µl RNase inhibitor (TAKARA), 50 ng/µl tRNA (Moniter, R., *et al., Biochim. Biophys. Acta.,* 43, p. 1- (1960)), and 0.46 µg/l creatine kinase (Roche), at final concentrations) containing 5.8 µl of the solution containing wheat germ extract prepared as described above in (2) was prepared. To this reaction solution, 2 or 8 µg of the mRNA containing the random sequence prepared as described in (1) was added, and the solution was incubated for 4 hours at 26°C.

A volume of 5 µl of each reaction solution was spotted onto filter paper, immediately after initiation of the reaction, 30 minutes after and 1, 2 and 4 hours after, and the incorporation of ¹⁴C-leu was measured by the solid support method using a liquid scintillation counter (LS6000IC: Beckman Coulter). The results are shown in Fig. 1. When the template activities using each mRNA were compared, the mRNA possessing the H base composition region, which excludes G, exhibited the highest template activity. Based on this result, those polynucleotides with the H base composition were used as candidate polynucleotides (random sequence) in the following experiments.

### Example 2: Screening for a nucleotide sequence having translation enhancement activity

### (1) Creation of RNA containing the candidate polynucleotide (random sequence)

PCR was carried out using as a template a plasmid into which a luciferase gene DNA (pSP-luc+: Promega, catalog NO: E1781) had been inserted, using a sense primer (SEQ ID NO: 5) containing a sequence having a 57 nts randomized site of the H base composition, an A to append a Kozak sequence to the 3' side thereof, a nucleotide sequence of the 5' end of the luciferase coding region on the 3' side thereof, a 12 nts consensus sequence linked on the 5' side of the randomized site, and a further SP6 promoter linked on the 5' side thereof, or a sense primer (SEQ ID NO: 3) containing a sequence having a 22 nts randomized site and the nucleotide sequence of the 5' end of the luciferase coding region on the 3' side thereof, a 12 nts consensus sequence linked on the 5' side of the randomized site, and a further SP6 promoter linked on the 5' side thereof, as well as an antisense primer (SEQ ID NO: 4) containing a sequence that is 3' downstream by 1652 bases from the stop codon of the luciferase gene DNA. The DNA fragment of approximately 3400 bp that was obtained was purified by ethanol precipitation, and was used as a template to perform transcription using SP6 RNA Polymerase (Promega). After phenol/chloroform extraction and ethanol precipitation of the RNA produced, this was purified with a Nick Column (Amersham Pharmacia Biotech). This was used as a translation template in the following experiment.

### (2) Protein synthesis with a cell-free protein synthesis system (batch method) using wheat germ extract

A volume of 25 µl of a reaction solution for protein synthesis (29 mM HEPES-KOH (pH 7.8), 95 mM potassium acetate, 2.7 mM magnesium acetate, 0.4 mM spermidine (Nacalai Techtonics), 0.23 mM each of the 20 types of L-type amino acids, 2.9 mM dithiothreitol, 1.2 mM ATP (Wako Pure Chemical), 0.25 mM GTP (Wako Pure Chemical), 15 mM creatine phosphate (Wako Pure Chemical), 0.9U/µl RNase inhibitor (TAKARA), 50ng/µl tRNA (Moniter, R., *et al., Biochim. Biophys. Acta.,* 43, p. 1- (1960)), and 0.46 µg/l creatine kinase (Roche), at final concentrations) containing 5.8 µl of the solution containing wheat germ extract prepared in Example 1(2) was made. To this reaction solution, 8 µg of the mRNA containing the random sequence created as described above in (1) was added, and the solution was incubated for 30 minutes at 26°C. After 30 minutes, cycloheximide (Wako Pure Chemical) was added so as to obtain a final concentration of 1.5 µM, to stop the protein synthesis.

### (3) Creation of a sucrose density gradient

A volume of 2.5 mL each of a 10% sucrose solution (25 mM Tris-HCl (pH 7.6), 50 mM potassium chloride, 5 mM magnesium chloride, 10% sucrose (Nacalai Techtonics), and 0.75 µM cycloheximide (Wako Pure Chemical), at final concentrations), and a 60% sucrose solution (25 mM Tris-HC1 (pH 7.6), 50 mM potassium chloride, 5 mM magnesium chloride, 60% sucrose (Nacalai Techtonics), and 0.75µM cycloheximide (Wako Pure Chemical), at final concentrations), was placed in a centrifuge tube, with the 60% sucrose solution as the lower layer and the 10% sucrose solution as the upper layer. Thereafter, a density gradient was made using a gradient maker (Towa Kagaku: BIOCOMP-GRADENT MASTER) with the following settings. (First time: SPEED: 25 RPM, ANGLE: 55°, TIME: 1 min. 50 sec.; Second time: SPEED: 25 RPM, ANGLE: 83.5°. TIME: 1min. 25 sec.). The density gradient solution produced was left to stand for 3 hours at 4°C.

### (4) Separation of polyribosomal fraction by sucrose density gradient centrifugation and extraction of RNA

To the reaction solution in which the protein synthesis described above in (2) had terminated, 75 µl of dilution solution (25 mM Tris-HCI (pH 7.6). 50 mM potassium chloride, 5 mM magnesium chloride (Nacalai Techtonics), at final concentrations) was added, and this was placed over the sucrose density gradient solution created as described above in (3), and centrifuged for 1 hour at 40,000 rpm, at 4°C (HITACHI: centrifuge CP65β, rotor P55ST2). Thereafter, fractions of 100 to 120 µl each were taken, and the optical density (O.D.) of each fraction was measured at 260 nm. The results are shown in Fig. 2. Using the AGPC method (Chomczynski, P., *et al.*, *Anal. Biochem.,* 162, 156-159 (1987)), RNA was extracted from the fractions 13 to 23 (sucrose concentration: 32.5% to 45%) in which polyribosomes were observed and in which protein synthesis was thought to proceed smoothly. To the total quantity of this extract, 25 U of DNase I (TAKARA) was added; the solution was incubated for 15 minutes at 37°C to degrade residual DNA; and thereafter, RNA was purified by phenol/chloroform extraction and ethanol precipitation.

### (5) Creation of cDNA and amplification

Using the RNA LA PCR Kit (AMV) ver1.1 (TAKARA), a reverse transcription reaction solution (5 mM magnesium chloride, 1×RNA PCR buffer, 1 mM dNTP mixture, 1.0 µM antisense primer (SEQ ID NO: 4), 1 U/µl RNase Inhibitor, and 0.25 U/µl Reverse Transcriptase, at final concentrations) was prepared; the entire quantity of the RNA extract as described above in (4) was added as a template; and a reverse transcription reaction was carried out to produce cDNA. In order to amplify this cDNA, PCR was performed using 1 µl of the reverse transcription product as a template, an oligonucleotide having a consensus sequence and the 3' end sequence of the SP6 promoter on the 5' side thereof as a sense primer (SEQ ID NO: 6), and an oligonucleotide containing a sequence approximately 60 bases downstream from the A of the start codon of the luciferase gene DNA as an antisense primer (SEQ ID NO: 7), to obtain a DNA fragment of approximately 150bp. After adding 5 U of Exonuclease I (USB) thereto and incubating it for 30 minutes at 37°C, incubation was performed for 30 minutes at 80°C to inactivate Exonuclease I. Thereafter, the entire quantity was recovered from the agarose gel using GFX^{™} PCR DNA and a Gel Band Purification Kit (Amersham Pharmacia Biotech).

### (6) Creation of translation template mRNA to be used in the next cycle

PCR was carried out using as a template a plasmid into which a luciferase gene DNA had been inserted, a sense primer (SEQ ID NO: 8) having a sequence that is complementary to the antisense primer as set forth in SEQ ID NO: 7 containing a sequence approximately 60 bases downstream from the A of the start codon of the luciferase gene DNA, and an antisense primer (SEQ ID NO: 9) containing a sequence that is further 3' downstream by 2 bases from the sequence as set forth in SEQ ID NO: 4. This produced a DNA fragment of approximately 3200 bp partially containing the luciferase gene DNA. PCR was performed again using two DNA fragments as templates, i.e., 1 µl of this PCR product and 1/50 of the quantity of the approximately 150 bp DNA fragment recovered as described above in (5), and using a sense primer (SEQ ID NO: 10) and an antisense primer (SEQ ID NO: 4). This produced a DNA fragment of approximately 3400 bp. With 3/4 of the amount of this product as a template, transcription was carried out using SP6 RNA Polymerase (Promega), and the RNA obtained was subjected to phenol/chloroform extraction and ethanol precipitation and then purified with a Nick Column (Amersham Pharmacia Biotech). Using this as a translation template and steps (2) to (6) in Example 2 as one cycle, steps (2) to (6) were repeated from the second cycle onward.

### Example 3: Examination of the template activity of the mRNA obtained after each cycle in Example 2 in a wheat germ cell-free protein synthesis system (batch method)

### (1) Protein synthesis with a wheat germ cell-free protein synthesis system (batch method)

A volume of 25 µl of a reaction solution for protein synthesis (29 mM HEPES-KOH (pH 7.8), 95 mM potassium acetate, 2.7 mM magnesium acetate, 0.4 mM spermidine (Nacalai Techtonics), 0.23 mM each of the 20 types of L-type amino acids, 2.9 mM dithiothreitol, 1.2 mM ATP (Wako Pure Chemical), 0.25 mM GTP (Wako Pure Chemical), 15 mM creatine phosphate (Wako Pure Chemical), 0.9 U/µl RNase inhibitor (TAKARA), 50 ng/µl tRNA (Moniter, R., *et al.*, *Biochim. Biophys. Acta.,* 43, p. 1- (1960)), 0.46 µg/l creatine kinase (Roche), and 2 nCi/µl ¹⁴C-Leu (Moravec), at final concentrations) containing 5.8 µl of the solution containing wheat germ extract prepared in Example 1(2) was prepared. To this reaction solution, 2 or 8 µg of the translation template mRNA was added, and these were incubated for 4 hours at 26°C.

For the mRNA that serves as a translation template, transcription was performed using the DNA fragment constructed in Example 2 (6) as a template, with SP6 RNA polymerase (Promega); RNA produced was subjected to phenol/chloroform extraction and ethanol precipitation, then purified with a Nick Column (Amersham Pharmacia Biotech), and used. Furthermore, as control sequences for translation efficiency enhancement, two kinds of DNA fragments containing the omega (Ω) sequence from the tobacco mosaic virus (TMV) in the 5' non-translated region, and either 0 nts or approximately 1600 nts in the 3' non-translated regions, were also transcribed and purified in the same manner, and used as controls.

A volume of 5 µl each of reaction solution was spotted onto filter paper immediately after initiation of the reaction, 30 minutes after and 1, 2 and 4 hours after, and the incorporation of ¹⁴C-leu was measured by the solid support method, using a liquid scintillation counter (LS6000IC: Beckman Coulter). The results are shown in Fig. 3. Template activity equivalent to those of the mRNA into which the Ω sequence was introduced was exhibited after four cycles when the mRNA with the 22 nts random site was used (Fig. 3A) and after three cycles when the 57 nts random site was used (Fig. 3B).

### Example 4: Examination of the effects of a novel sequence in a wheat germ cell-free protein synthesis system (batch method and dialysis method)

### (1) TA cloning and sequencing

Based on Example 3, DNA fragments obtained in Example 2 (6), after 4 cycles using the mRNA with the 22 nts random site, and after 3 cycles using the mRNA with the 57 nts random site, were added to the reaction solution (1 × Rapid Ligation Buffer, 5 ng/µl pGEM-T Easy Vector, at final concentrations) using the pGEM-T Easy Kit (Promega) and incubated for 4 hours at 14°C to integrate the DNA fragments into the pGEM-T Easy Vector. Thereafter, the entire quantity was used to transform *Escherichia coli* JM109 (TAKARA); plasmids were extracted from the colonies produced; and the sequence that was inserted into the plasmid was sequenced. As a result, novel sequences were identified: 27 types (SEQ ID NO: 11 to 37) for the 57 nt randomization and 96 types (SEQ ID NO: 40 to 135) for the 22 nt randomization. In addition, the DNA fragments with the 57 nt randomization, obtained after two cycles, were cloned in the same manner, and with a portion thereof, the inserted sequence was sequenced. As a result, novel sequences as shown in SEQ ID NO: 38 and 39 were identified.

### (2) Construction of a DNA fragment containing a novel sequence

PCR was performed, using as a template a plasmid into which luciferase gene DNA (pSP-luc+: Promega, catalog NO: E1781) was inserted, using a sense primer (No. 57-6: SEQ ID NO: 136, No. 57-40: SEQ ID NO: 137, No. 57-91: SEQ ID NO: 138, No. 22-2: SEQ ID NO: 139, No. 22-5: SEQ ID NO: 140, No. 22-10: SEQ ID NO: 141, No. 22-12: SEQ ID NO: 142, No. 22-18: SEQ ID NO: 143, and No. 22-23: SEQ ID NO: 144) designed to link a nucleotide sequence obtained in (1) (No. 57-6: SEQ ID NO: 11, No. 57-40: SEQ ID NO: 15 and No. 57-91: SEQ ID NO: 20 when the random site was 57 nts, and No. 22-2: SEQ ID NO: 41, No. 22-5: SEQ ID NO: 44, No. 22-10: SEQ ID NO: 49, No. 22-12: SEQ ID NO: 51, No. 22-18: SEQ ID NO: 57 and No. 22-23: SEQ ID NO: 62 when the random site was 22 nts, respectively), a consensus sequence on the 5' side thereof and a sequence containing the start codon of luciferase on the 3' side thereof (for No. 57-6, No. 57-40 and No. 57-91, a further KOZAK A on the 5' side of the start codon), and an antisense primer as set forth in SEQ ID NO: 4, so as to have a 3' non-translated region of approximately 1600 nts. PCR was performed, using as a template 1 µl of the approximately 3400 bp DNA fragment (approximately 1600 nts of 3' non-translated region) that was obtained, using a sense primer (SEQ ID NO: 145) having a sequence in which an SP6 promoter sequence was linked on the 5' side of the consensus sequence and the antisense primer set forth in SEQ ID NO: 4, to once again produce a DNA fragment of approximately 3400 bp. This was used as a translation template in the following experiment.

### (3) Protein synthesis with a wheat germ cell-free protein synthesis system (batch method)

A volume of 25 µl of a reaction solution for protein synthesis (29 mM HEPES-KOH (pH 7.8), 95 mM potassium acetate, 2.7 mM magnesium acetate, 0.4 mM spermidine (Nacalai Techtonics), 0.23 mM each of the 20 types of L-type amino acids, 2.9 mM dithiothreitol, 1.2 mM ATP (Wako Pure Chemical), 0.25 mM GTP (Wako Pure Chemical), 15 mM creatine phosphate (Wako Pure Chemical), 0.9 U/µl RNase inhibitor (TAKARA), 50 ng/µl tRNA (Moniter, R., *et al., Biochim. Biophys. Acta.,* 43, p. 1- (1960)), 0.46 µg/l creatine kinase (Roche), and 2 nCi/µl ¹⁴C-leu (Moravec), at final concentrations) containing 5.8 µl of the solution containing wheat germ extract prepared in Example 1(2) was prepared. To this reaction solution, 2 or 8 µg of the translation template mRNA was added, and these were incubated for 4 hours at 26°C.

For the mRNA to be used as a translation template, transcription was carried out using the DNA synthesized as described above in (2) as a template, and using SP6 RNA polymerase (Promega), the RNA produced was subjected to phenol/chloroform extraction and ethanol precipitation, and then purified with a Nick Column (Amersham Pharmacia Biotech), and used. In addition, as control sequences for translation efficiency enhancement, two kinds of DNA fragments containing the omega (Ω) sequence from the tobacco mosaic virus (TMV) in the 5' non-translated region, and either 0 nts or approximately 1600 nts in the 3' non-translated regions, were transcribed and purified in the same manner, and used as controls.

A volume of 5 µl each of reaction solution was spotted onto filter paper immediately after initiation of the reaction, 30 minutes after, and 1, 2 and 4 hours after, and the incorporation of ¹⁴C-leu was measured by the solid support method, using a liquid scintillation counter (LS6000IC: Beckman Coulter). The results are shown in Fig. 4. The synthesis system using RNAs containing sequences No. 57-6 (Fig. 4B) and No. 22-12 (Fig. 4A) in the mRNAs containing a 3' non-translated region, was capable of producing the same quantity of target protein as in the case of RNAs containing the Ω sequence. From this, it is thought that the other sequences that were obtained demonstrate an equal or greater translation template activity.

### (4) Protein synthesis with a wheat germ cell-free protein synthesis system (dialysis)

A volume of 50 µl of a reaction solution for protein synthesis (29 mM HEPES-KOH (pH 7.8), 95 mM potassium acetate, 2.7 mM magnesium acetate, 0.4 mM spermidine (Nacalai Techtonics), 0.23 mM each of the 20 types of L-type amino acids, 2.9 mM dithiothreitol, 1.2 mM ATP (Wako Pure Chemical), 0.25 mM GTP (Wako Pure Chemical), 15 mM creatine phosphate (Wako Pure Chemical), 0.9 U/µl RNase inhibitor (TAKARA), 50 ng/µl tRNA (Moniter, R., *et al*., *Biochim. Biophys. Acta.,* 43, p. 1- (1960)), and 0.46 µg/l creatine kinase (Roche), at final concentrations) containing 11.6 µl of the solution containing wheat germ extract prepared in Example 1(2) was prepared. To this reaction solution, 16 µg/50 µl of the translation template mRNA was added, and this was incubated for 48 hours at 26°C.

For the mRNA that serves as a translation template, transcription was performed using the SP6 RNA polymerase (Promega) and using as a template a circular plasmid DNA in which, based on the pEU-GFP vector (Sawasaki, T., *et al., PNAS,* 99 (23), pp. 14652-7 (2002)) into which the GFP gene DNA (Chiu, W. L., *et al., Curr. Biol.* 6, pp. 325-330 (1996)) had been inserted, the Ω sequence portion had been replaced with sequence No. 57-6. The RNA produced was subjected to phenol/chloroform extraction and ethanol precipitation, and then purified with a Nick Column (Amersham Pharmacia Biotech) and used. In addition, as a control sequence for translation efficiency enhancement, a DNA fragment containing the omega (Ω) sequence from the tobacco mosaic virus (TMV) in the 5' non-translated region, and having a 3' non-translated region of approximately 1600 nts was transcribed, purified, and used as a control.

Every 24 and 48 hours, 0.5 µl was collected from the reaction solution of each of the synthesis systems described above, and this was subjected to separation by 12.5% SDS-polyacrylamide electrophoresis (SDS-PAGE), and analyzed by staining with Coomassie Brilliant Blue (CBB). The results are shown in Fig. 5. As in the batch method, the dialysis system using RNA containing sequence No. 57-6 in the mRNA containing a 3' non-translated region, demonstrated the same level of translation template activity as in the case of RNAs containing the Ω sequence. From this, it is thought that the other sequences that were obtained demonstrate an equal or greater translation template activity.

### Possibilities for Industrial Use

By virtue of the present invention, a nucleotide sequence that is an artificial sequence which does not naturally exist and has the activity of regulating translation efficiency, and a polynucleotide that comprises the nucleotide sequence are provided. By using the polynucleotide, protein synthesis can be carried out in a protein synthesis system with extremely high efficiency.

The present application is based on Japanese Patent Application 2001-396941 filed in Japan, the entire contents of which are incorporated herein.

In addition, literatures including the patents and the patent applications quoted in the present specification are incorporated herein by reference in its entirety in the same way as if the content thereof were disclosed.

## Claims

1. A polynucleotide having translation enhancement activity, comprising the nucleotide sequence set forth in SEQ ID NO: 11.

2. The polynucleotide according to claim 1, which comprises the nucleotide sequence set forth in SEQ ID NO: 136.

3. A nucleic acid molecule appropriate for translation of a polynucleotide coding for a target polypeptide, comprising the polynucleotide according to any of claims 1 or 2 upstream of the said coding polynudeotide.

4. The nucleic acid molecule according to claim 3, further comprising a promoter sequence upstream of the polynucleotide according to any of claims 1 or 2.

5. The nucleic acid molecule according to claim 4, further comprising a 3' non-translated region downstream of the said coding polynucleotide.

6. A nucleic acid molecule having translation enhancement activity obtainable by transcribing the nucleic acid molecule according to any of claims 4 or 5.

7. A vector comprising the polynucleotide according to any of claims 1 or 2 and/or the nucleic acid molecule according to any of claims 3 to 5.

8. A protein synthesis method **characterised by** the use of the nucleic acid molecule according to any of claims 3 to 5 or the vector according to claim 7 as a template for transcription and/or translation.

9. A protein synthesis method **characterised by** the use of the nucleic acid molecule according to claim 6 as a template for translation.

## Patentansprüche

1. Polynucleotid mit Translationssteigerungsaktivität, umfassend das Nucleotid wie in SEQ ID NO: 11 gezeigt.

2. Polynucleotid nach Anspruch 1, das die Nucleotidsequenz wie in SEQ ID NO: 136 gezeigt umfasst.

3. Nucleinsäuremolekül, das geeignet ist für die Translation eines Polynucleotids, das ein Zielpolypeptid codiert, umfassend das Polynucleotid nach einem der Ansprüche 1 oder 2 stromaufwärts des codierenden Polynucleotids.

4. Nucleinsäuremolekül nach Anspruch 3, weiter umfassend eine Promotorsequenz stromaufwärts des Polynucleotids nach einem der Ansprüche 1 oder 2.

5. Nucleinsäuremolekül nach Anspruch 4, weiter umfassend eine 3'-nicht-translatierte Region stromabwärts des codierenden Polynucleotids.

6. Nucleinsäuremolekül mit Translationssteigerungsaktivität, erhältlich durch Transkribieren des Nucleinsäuremoleküls nach einem der Ansprüche 4 oder 5.

7. Vektor, umfassend das Polynucleotid nach einem der Ansprüche 1 oder 2 und/oder das Nucleinsäuremolekül nach einem der Ansprüche 3 bis 5.

8. Proteinsyntheseverfahren, **gekennzeichnet durch** die Verwendung des Nucleinsäuremoleküls nach einem der Ansprüche 3 bis 5 oder des Vektors nach Anspruch 7 als Matrize für Transkription und/oder Translation.

9. Proteinsyntheseverfahren, **gekennzeichnet durch** die Verwendung des Nucleinsäuremoleküls nach Anspruch 6 als Matrize für die Translation.

## Revendications

1. Polynucléotide ayant une activité d'amélioration de traduction, comprenant la séquence de nucléotides présentée dans SEQ ID n°11.

2. Polynucléotide selon la revendication 1, qui comprend la séquence de nucléotides présentée dans SEQ ID n° 136.

3. Molécule d'acide nucléique appropriée pour la traduction d'un polynucléotide codant un polypeptide cible, comprenant le polynucléotide selon l'une quelconque des revendications 1 ou 2 en amont dudit polynucléotide codant.

4. Molécule d'acide nucléique selon la revendication 3, comprenant en outre une séquence promotrice en amont du polynucléotide selon l'une quelconque des revendications 1 ou 2.

5. Molécule d'acide nucléique selon la revendication 4, comprenant en outre une région 3' non traduite en aval dudit polynucléotide codant.

6. Molécule d'acide nucléique ayant une activité d'amélioration de traduction obtenue par transcription de la molécule d'acide nucléique selon l'une quelconque des revendications 4 ou 5.

7. Vecteur comprenant le polynucléotide selon l'une quelconque des revendications 1 ou 2 et/ou la molécule d'acide nucléique selon l'une quelconque des revendications 3 à 5.

8. Procédé de synthèse de protéines **caractérisé par** l'utilisation de la molécule d'acide nucléique selon l'une quelconque des revendications 3 à 5 ou du vecteur selon la revendication 7 comme matrice de transcription et/ou de traduction.

9. Procédé de synthèse de protéines **caractérisé par** l'utilisation de la molécule d'acide nucléique selon la revendication 6 comme matrice de traduction.


### SEQUENCE LISTING

<110> ENDO, Yaeta
<120> Nucleotide Sequence Having Activity Regulating Translation Efficiency and Use Thereof
<130> 09522
<150> JP 2001-396941
<151> 2001-12-27
<160> 145
<170> PatentIn version 3.1
<210> 1
<211> 76
<212> DNA
<213> Artificial
<220>
<221> misc_feature
   <223> Synthetic DNA.
<400> 1
   cgatttaggt gacactatag aactcaccta tctcbbbbbb bbbbbbbbbb bbbbbbatgg 60
   aagacgcca aaaacat 76
<210> 2
<211> 76
<212> DNA
<213> Artificial
<220>
<221> misc_feature
   <223> Synthetic DNA.
<400> 2
   cgatttaggt gacactatag aactcaccta tctcvvvvvv vvvvvvvvvv vvvvvvatgg 60
   aagacgcca aaaacat 76
<210> 3
<211> 76
<212> DNA
<213> Artificial
<220>
<221> misc_feature
   <223> Synthetic DNA.
<400> 3
   cgatttaggt gacactatag aactcaccta tctchhhhhh hhhhhhhhhh hhhhhhatgg 60
   aagacgccaa aaacat 76
<210> 4
<211> 20
<212> DNA
<213> Artificial
<220>
   <221> misc_feature
   <223> Synthetic DNA.
<400> 4
   gtcagacccc gtagaaaaga 20
<210> 5
<211> 112
<212> DNA
<213> Artificial
<220>
<221> misc_feature
   <223> Synthetic DNA.
<400> 5
   cgatttaggt gacactatag aactcaccta tctchhhhhh hhhhhhhhhh hhhhhhhhhh 60
   hhhhhhhhhh hhhhhhhhhh hhhhhhhhhh haatggaaga cgccaaaaac at 112
<210> 6
<211> 27
<212> DNA
<213> Artificial
<220>
<221> misc_feature
   <223> Synthetic DNA.
<400> 6
   ggtgacacta tagaactcac ctatctc 27
<210> 7
<211> 20
<212> DNA
<213> Artificial
<220>
<221> misc_feature
   <223> Synthetic DNA
<400> 7
   tatgcagttg ctctccagcg 20
<210> 8
<211> 20
<212> DNA
<213> Artificial
<220>
<221> mise_feature
   <223> Synthetic DNA.
<400> 8
   ggagagcaac tgcataaggc 20
<210> 9
<211> 20
<212> DNA
<213> Artificial
<220>
<221> misc_feature
   <223> Synthetic DNA.
<400> 9
   agcgtcagac cccgtagaaa 20
<210> 10
<211> 21
<212> DNA
<213> Artificial
<220>
<221> misc_feature
   <223> Synthetic DNA.
<400> 10
   gcgtagcatt taggtgacac t 21
<210> 11
   <211> 57
   <212> DNA
   <213> Artificial
<220>
   <221> misc_feature
   <223> Synthetic DNA.
<400> 11
   cccaacacct aataacattc aatcactctt tccactaacc acctatctac atcacca 57
<210> 12
   <211> 57
   <212> DNA
   <213> Artificial
<220>
   <221> mise_feature
   <223> Synthetic DNA.
<400> 12
   ataccactca atcccacact cacaccatcc cacacatccc ccaccccatt ttctcca 57
<210> 13
   <211> 57
   <212> DNA
   <213>. Artificial
<220>
   <221> misc_feature
   <223> Synthetic DNA.
<400> 13
   ccaccacatt catccatcct ctactcacta tatcaacccc tccacttacc tctccac 57
<210> 14
   <211> 57
   <212> DNA
   <213> Artificial
<220>
   <221> misc_feature
   <223> Synthetic DNA.
<400> 14
   cctaacacta cacaaaccta tcaattcata ttttctaccc tcactcactc actccca 57
<210> 15
   <211> 57
   <212> DNA
   <213> Artificial
<220>
   <221> misc_feature
   <223> Synthetic DNA.
<400> 15
   ctataaaccc accttaccaa tctccacatt caatatctct ccccttaccc tcatcac 57
<210> 16
   <211> 57
   <212> DNA
   <213> Artificial
<220>
   <221> misc_feature
   <223> Synthetic DNA.
<400> 16
   atctcaatac tacatctaac accaaacatc ctcccatcca cccataacac tccacct 57
<210> 17
   <211> 57
   <212> DNA
   <213> Artificial
<220>
   <221> misc_feature
   <223> Synthetic DNA.
<400> 17
   tacccacatt caccactctc actaatatat taaccaatcc tattaaaaca acccacc 57
<210> 18
   <211> 57
   <212> DNA
   <213> Artificial
<220>
   <221> misc_feature
   <223> Synthetic DNA.
<400> 18
   ctctactcac catttacctc caactcttcc ctacaatcta cccatcccct tcattat 57
<210> 19
   <211> 57
   <212> DNA
   <213> Artificial
<220>
   <221> misc_feature
   <223> Synthetic DNA.
<400> 19
   ccccccccct tacaattcca caaacacttt ctccttctat ctacctacaa atacttc 57
<210> 20
   <211> 57
   <212> DNA
   <213> Artificial
<220>
   <221> misc_feature
   <223> Synthetic DNA.
<400> 20
   ccaacaccaa taccaactcc actcacctat ctccacctca cacacacttt tccatcc 57
<210> 21
   <211> 57
   <212> DNA
   <213> Artificial
<220>
   <221> misc_feature
   <223> Synthetic DNA.
<400> 21
   tcttccacct tatcccaccc acatccaatg cacataaaca ttcctcccat tttttct 57
<210> 22
   <211> 57
   <212> DNA
   <213> Artificial
<220>
   <221> misc_feature
   <223> Synthetic DNA.
<400> 22
   cccagtccca aaccacttca atttccttcc caccatccta accaattacc attaccc 57
<210> 23
   <211> 57
   <212> DNA
   <213> Artificial
<220>
   <221> misc_feature
   <223> Synthetic DNA.
<400> 23
   aactcaccat caaccaccct tcaacacccc atcttccctt accactactc taccaca 57
<210> 24
   <211> 57
   <212> DNA
   <213> Artificial
<220>
   <221> misc_feature
   <223> Synthetic DNA.
<400> 24
   tccacaacaa caccctcaca ccccatcata atctaatcta catttccata tttcaca 57
<210> 25
   <211> 57
   <212> DNA
   <213> Artificial
<220>
   <221> misc_feature
   <223> Synthetic DNA.
<400> 25
   aacccaccat ttatcccaac cttccccacc acacatatca tatctacatc taccctc 57
<210> 26
   <211> 57
   <212> DNA
   <213> Artificial
<220>
   <221> misc_feature
   <223> Synthetic DNA.
<400> 26
   cccacaacaa caccctcaca ccccatcata atctaatcta catttccata tttcaca 57
<210> 27
<211> 57
   <212> DNA
   <213> Artificial
<220>
   <221> misc_feature
   <223> Synthetic DNA.
<400> 27
   ccccacataa tctacaaccc ccctcacacc atcaacactc aatcaataac ccaacat 57
<210> 28
   <211> 57
   <212> DNA
   <213> Artificial
<220>
   <221> misc_feature
   <223> Synthetic DNA.
<400> 28
   ccatcaccat ccacttaact tatccaacca taccaccccc cctatcctac cactccc 57
<210> 29
   <211> 57
   <212> DNA
   <213> Artificial
<220>
   <221> misc_feature
   <223> Synthetic DNA.
<400> 29
   cccacaacaa caccctcaca ccccgtcata atctaatcta catttccata tttcaca 57
<210> 30
   <211> 57
   <212> DNA
   <213> Artificial
<220>
   <221> misc_feature
   <223> Synthetic DNA.
<400> 30
   ccactaccac ttaatctaaa actcacctaa tcaaaatcct catacctttc ccacttc 57
<210> 31
   <211> 57
   <212> DNA
   <213> Artificial
<220>
   <221> misc_feature
   <223> Synthetic DNA.
<400> 31
   aacccaccat ttatcccaac cttccccacc acacatatca tatctacatc tactctc 57
<210> 32
   <211> 57
   <212> DNA
   <213> Artificial
<220>
   <221> misc_feature
   <223> Synthetic DNA.
<220>
   <221> misc_feature
   <222> (3).. (3)
   <223> "n" stands for a. g, c or t.
<400> 32
   ccntcaccat ccacttaact tatccaacca taccaccccc cctatcctac cactccc 57
<210> 33
   <211> 57
   <212> DNA
   <213> Artificial
<220>
   <221> misc_feature
   <223> Synthetic DNA.
<400> 33
   caccccacta tcctaatcaa cctctaacta cataccacta cctatttatc catacac 57
<210> 34
   <211> 57
   <212> DNA
   <213> Artificial
<220>
   <221> misc_feature
   <223> Synthetic DNA.
<400> 34
   cccacaacaa caccctcaca ccccatcata atctaatcta catttccata tttcaca 57
<210> 35
   <211> 57
   <212> DNA
   <213> Artificial
<220>
   <221> misc_feature
   <223> Synthetic DNA.
<400> 35
   cccacaacaa caccctcaca ccccatcata atctaatcta catttccata tttcaca 57
<210> 36
   <211> 57
   <212> DNA
   <213> Artificial
<220>
   <221> misc_feature
   <223> Synthetic DNA.
<400> 36
   acaccactac cacacccccc cttaatttac aactcacctc ctactcccac aaccaac 57
<210> 37
   <211 > 57
   <212> DNA
   <213> Artificial
<220>
   <221> misc_feature
   <223> Synthetic DNA.
<400> 37
   cacatcctaa ttcttacata acccacatta ccctacatct taatcccaca ttctcac 57
<210> 38
<211> 57
   <212> DNA
   <213> Artificial
<220>
   <221> misc_feature
   <223> Synthetic DNA.
<400> 38
   tcatcctcaa cccacctcct atatatccca attttctcaa tcctccccct tttaata 57
<210> 39
   <211> 57
   <212> DNA
   <213> Artificial
<220>
   <221> misc_feature
   <223> Synthetic DNA.
<400> 39
   tcacctcccc actccccaac ccaataacat aaacccccaa ccataaaaac tccactt 57
<210> 40
<211> 22
   <212> DNA
   <213> Artificial
<220>
   <221> misc_feature
   <223> Synthetic DNA.
<400> 40
   tccctactac cccttaactc tc 22
<210> 41
   <211> 22
   <212> DNA
   <213> Artificial
<220>
   <221> misc_feature
   <223> Synthetic DNA.
<400> 41
   cttatcctat tttcctctta ca 22
<210> 42
   <211> 22
   <212> DNA
   <213> Artificial
<220>
   <221> mise_feature
   <223> Synthetic DNA.
<400> 42
   cttttctttc attccttaac tt 22
<210> 43
   <211> 22
   <212> DNA
   <213> Artificial
<220>
   <221> misc_feature
   <223> Synthetic DNA.
<400> 43
   cctttcaaaa ctcattaatt tc 22
<210> 44
   <211> 22
   <212> DNA
   <213> Artificial
<220>
   <221> misc_feature
   <223> Synthetic DNA.
<400> 44
   tcctatccaa ccatacatcc tt 22
<210> 45
   <211> 22
   <212> DNA
   <213> Artificial
<220>
   <221> misc_feature
   <223> Synthetic DNA.
<400> 45
   tcaattttcc accacactac tc 22
<210> 46
   <211> 22
   <212> DNA
   <213> Artificial
<220>
   <221> mise_feature
   <223> Synthetic DNA.
<400> 46
   ttaatattcc tcacattctc ta 22
<210> 47
   <211> 22
   <212> DNA
   <213> Artificial
<220>
   <221> misc_feature
   <223> Synthetic DNA.
<400> 47
   tctcacaata tttataacaa tt 22
<210> 48
   <211> 22
   <212> DNA
   <213> Artificial
<220>
   <221> misc_feature
   <223> Synthetic DNA
<400> 48
   ttttcatcaa cactaactat cc 22
<210> 49
   <211> 22
   <212> DNA
   <213> Artificial
<220>
   <221> misc_feature
   <223> Synthetic DNA.
<400> 49
   tcccacattc ccccctatct ct 22
<210> 50
   <211> 22
   <212> DNA
   <213> Artificial
<220>
   <221> misc_feature
   <223> Synthetic DNA.
<400> 50
   ctttttttac tcctccaccc ct 22
<210> 51
   <211> 22
   <212> DNA
   <213> Artificial
<220>
   <221> misc_feature
   <223> Synthetic DNA.
<400> 51
   attttttctt aattccctca tt 22
<210> 52
   <211> 22
   <212> DNA
   <213> Artificial
<220>
   <221> misc_feature
   <223> Synthetic DNA.
<400> 52
   tcacatctat taatctattc ac 22
<210> 53
   <211> 22
   <212> DNA
   <213> Artificial
<220>
   <221> misc_feature
   <223> Synthetic DNA.
<400> 53
   atttttccat ataaccttct ct 22
<210> 54
   <211> 22
   <212> DNA
   <213> Artificial
<220>
   <221> misc_feature
   <223> Synthetic DNA.
<400> 54
   ctttcattac cataaaatcc tt 22
<210> 55
   <211> 22
   <212> DNA
   <213> Artificial
<220>
   <221> misc_feature
   <223> Synthetic DNA.
<400> 55
   ctcatttcaa attttcttac ca 22
<210> 56
   <211> 22
   <212> DNA
   <213> Artificial
<220>
   <221> misc_feature
   <223> Synthetic DNA.
<400> 56
   attccattcc ctaattttca at 22
<210> 57
   <211> 22
   <212> DNA
   <213> Artificial
<220>
   <221> misc_feature
   <223> Synthetic DNA.
<400> 57
   tctccacttt tcttttacac cc 22
<210> 58
   <211> 22
   <212> DNA
   <213> Artificial
<220>
   <221> misc_feature
   <223> Synthetic DNA.
<400> 58
   caaatcttta attcttccct ac 22
<210> 59
   <211> 22
   <212> DNA
   <213> Artificial
<220>
   <221> misc_feature
   <223> Synthetic DNA.
<400> 59
   attttttctt aattttccat tc 22
<210> 60
   <211> 22
   <212> DNA
   <213> Artificial
<220>
   <221> misc_feature
   <223> Synthetic DNA.
<400> 60
   aaccaataat ccatcctttt ta 22
<210> 61
   <211> 22
   <212> DNA
   <213> Artificial
<220>
   <221> misc_feature
   <223> Synthetic DNA.
<400> 61
   cttttcacta ctttacttct tt 22
<210> 62
   <211> 22
   <212> DNA
   <213> Artificial
<220>
   <221> misc_feature
   <223> Synthetic DNA.
<400> 62
   acactatcaa tacctactct tt 22
<210> 63
   <211> 22
   <212> DNA
   <213> Artificial
<220>
   <221> misc_feature
   <223> Synthetic DNA.
<400> 63
   taacacttat tcaataattc aa 22
<210> 64
   <211> 22
   <212> DNA
   <213> Artificial
<220>
   <221> misc_feature
   <223> Synthetic DNA.
<400> 64
   acttattttt ccacacttac tt 22
<210> 65
   <211> 22
   <212> DNA
   <213> Artificial
<220>
   <221> misc_feature
   <223> Synthetic DNA.
<400> 65
   tttactattc tttctattct tt 22
<210> 66
   <211> 22
   <212> DNA
   <213> Artificial
<220>
   <221> misc_feature
   <223> Synthetic DNA.
<400> 66
   tcattttacc aatcatccct ta 22
<210> 67
   <211> 22
   <212> DNA
   <213> Artificial
<220>
   <221> misc_feature
   <223> Synthetic DNA.
<400> 67
   tcttaaccaa tttcatacca cc 22
<210> 68
   <211> 22
   <212> DNA
   <213> Artificial
<220>
   <221> misc_feature
   <223> Synthetic DNA.
<400> 68
   tacacataca atctaattcc ct 22
<210> 69
   <211> 22
   <212> DNA
   <213> Artificial
<220>
   <221> misc_feature
   <223> Synthetic DNA.
<400> 69
   acacatctat tatccctctt ct 22
<210> 70
   <211> 22
   <212> DNA
   <213> Artificial
<220>
   <221> misc_feature
   <223> Synthetic DNA.
<400> 70
   ctacctccat ttcaaccata tt 22
<210> 71
   <211> 22
   <212> DNA
   <213> Artificial
<220>
   <221> misc_feature
   <223> Synthetic DNA.
<400> 71
   tctctatatt ttcaataaca ac 22
<210> 72
   <211> 22
   <212> DNA
   <213> Artificial
<220>
   <221> misc_feature
   <223> Synthetic DNA.
<400> 72
   acattaacac ttttttttaa cc 22
<210> 73
   <211> 22
   <212> DNA
   <213> Artificial
<220>
   <221> misc_feature
   <223> Synthetic DNA.
<400> 73
   tcaatcccct ttcataccaa tt 22
<210> 74
   <211> 22
   <212> DNA
   <213> Artificial
<220>
   <221> misc_feature
   <223> Synthetic DNA.
<400> 74
   tactctttta actcctattc ta 22
<210> 75
   <211> 22
   <212> DNA
   <213> Artificial
<220>
   <221> misc_feature
   <223> Synthetic DNA.
<400> 75
   tcacattatc ttttctcttt tc 22
<210> 76
   <211> 22
   <212> DNA
   <213> Artificial
<220>
   <221> misc_feature
   <223> Synthetic DNA.
<400> 76
   ctaccttacc aattttttac cc 22
<210> 77
   <211> 22
   <212> DNA
   <213> Artificial
<220>
   <221> misc_feature
   <223> Synthetic DNA.
<400> 77
   gccttacccc ctcatcccct ca 22
<210> 78
   <211> 22
   <212> DNA
   <213> Artificial
<220>
   <221> misc_feature
   <223> Synthetic DNA.
<400> 78
   tattcacatc accccttaac tt 22
<210> 79
   <211> 22
   <212> DNA
   <213> Artificial
<220>
   <221> misc_feature
   <223> Synthetic DNA.
<400> 79
   tctcaacaac atactttttt ta 22
<210> 80
   <211> 22
   <212> DNA
   <213> Artificial
<220>
   <221> misc_feature
   <223> Synthetic DNA.
<400> 80
   cctactactt tccaatcttt tc 22
<210> 81
   <211> 22
   <212> DNA
   <213> Artificial
<220>
   <221> misc_feature
   <223> Synthetic DNA.
<400> 81
   ttttatattc aacatactat tc 22
<210> 82
   <211> 22
   <212> DNA
   <213> Artificial
<220>
   <221> misc_feature
   <223> Synthetic DNA.
<400> 82
   tctttcactt aaactatcca tt 22
<210> 83
   <211> 22
   <212> DNA
   <213> Artificial
<220>
   <221> misc_feature
   <223> Synthetic DNA.
<400> 83
   caccacccac acacatacaa ca 22
<210> 84
   <211> 22
   <212> DNA
   <213> Artificial
<220>
   <221> misc_feature
   <223> Synthetic DNA.
<400> 84
   acattctcca tacctacatt tc 22
<210> 85
   <211> 22
   <212> DNA
   <213> Artificial
<220>
   <221> mise_feature
   <223> Synthetic DNA.
<400> 85
   ccctaactca atcatcatac at 22
<210> 86
   <211> 22
   <212> DNA
   <213> Artificial
<220>
   <221> misc_feature
   <223> Synthetic DNA.
<400> 86
   aatccccttt tcacaaacct tt 22
<210> 87
   <211> 22
   <212> DNA.
   <213> Artificial
<220>
   <221> misc_feature
   <223> Synthetic DNA.
<400> 87
   aatccccttt tcacaaacct tt 22
<210> 88
   <211> 22
   <212> DNA
   <213> Artificial
<220>
   <221> misc_feature
   <223> Synthetic DNA.
<400> 88
   aaccttcttc taaatccatc at 22
<210> 89
   <211> 22
   <212> DNA
   <213> Artificial
<220>
   <221> misc_feature
   <223> Synthetic DNA.
<400> 89
   tacattcaca cttaatttat cc 22
<210> 90
   <211> 22
   <212> DNA
   <213> Artificial
<220>
   <221> misc_feature
   <223> Synthetic DNA.
<400> 90
   tacttattta accctattca cc 22
<210> 91
   <211> 22
   <212> DNA
   <213> Artificial
<220>
   <221> misc_feature
   <223> Synthetic DNA.
<400> 91
   tcatactacc aaaaacctat ca 22
<210> 92
   <211> 22
   <212> DNA
   <213> Artificial
<220>
   <221> misc_feature
   <223> Synthetic DNA.
<400> 92
   tcattatcac attacactta ct 22
<210> 93
   <211> 22
   <212> DNA
   <213> Artificial
<220>
   <221> misc_feature
   <223> Synthetic DNA.
<400> 93
   tcaatatttc cctctctaaa at 22
<210> 94
   <211> 22
   <212> DNA
   <213> Artificial
<220>
   <221> misc_feature
   <223> Synthetic DNA.
<400> 94
   ttctatcatt ttctacttat ta 22
<210> 95
   <211> 22
   <212> DNA
   <213> Artificial
<220>
   <221> misc_feature
   <223> Synthetic DNA.
<400> 95
   atattattaa cccttttcaa at 22
<210> 96
   <211> 22
   <212> DNA
   <213> Artificial
<220>
   <221> misc_feature
   <223> Synthetic DNA.
<400> 96
   ctaacttcta cacaacattt tc 22
<210> 97
   <211> 22
   <212> DNA
   <213> Artificial
<220>
   <221> misc_feature
   <223> Synthetic DNA.
<400> 97
   tactatctac cctcacacca ct 22
<210> 98
   <211> 22
   <212> DNA
   <213> Artificial
<220>
   <221> misc_feature
   <223> Synthetic DNA.
<400> 98
   accaaaccaa tttaattttt tc 22
<210> 99
   <211> 22
   <212> DNA
   <213> Artificial
<220>
   <221> misc feature
   <223> Synthetic DNA.
<400> 99
   tatatttccc ataattacaa aa 22
<210> 100
   <211> 22
   <212> DNA
   <213> Artificial
<220>
   <221> misc_feature
   <223> Synthetic DNA.
<400> 100
   aacatttttt catcttttca ta 22
<210> 101
   <211> 22
   <212> DNA
   <213> Artificial
<220>
   <221> misc_feature
   <223> Synthetic DNA.
<400> 101
   actctcacct tcaaccccct tt 22
<210> 102
   <211> 22
   <212> DNA
   <213> Artificial
<220>
   <221> misc_feature
   <223> Synthetic DNA.
<400> 102
   tctctcatcc cacctcaatt tt 22
<210> 103
   <211> 22
   <212> DNA
   <213> Artificial
<220>
   <221> misc_feature
   <223> Synthetic DNA.
<400> 103
   attctcttat catacacaca cc 22
<210> 104
   <211> 22
   <212> DNA
   <213> Artificial
<220>
   <221> misc_feature
   <223> Synthetic DNA.
<400> 104
   tcactttttc caccacaatc ac 22
<210> 105
   <211> 22
   <212> DNA
   <213> Artificial
<220>
   <221> misc_feature
   <223> Synthetic DNA.
<400> 105
   tcttttaaaa ctttcctcaa tc 22
<210> 106
   <211> 22
   <212> DNA
   <213> Artificial
<220>
   <221> misc_feature
   <223> Synthetic DNA.
<400> 106
   tatttttcaa ccctatatta ta 22
<210> 107
   <211> 22
   <212> DNA
   <213> Artificial
<220>
   <221> misc_feature
   <223> Synthetic DNA.
<400> 107
   ctatccttta aactctaacc tc 22
<210> 108
   <211> 22
   <212> DNA
   <213> Artificial
<220>
   <221> misc_feature
   <223> Synthetic DNA.
<400> 108
   taaacttttc cttccctcta ct 22
<210> 109
   <211> 22
   <212> DNA
   <213> Artificial
<220>
   <221> misc_feature
   <223> Synthetic DNA.
<400> 109
   tatttcctca atttatctct ct 22
<210> 110
   <211> 22
   <212> DNA
   <213> Artificial
<220>
   <221> misc_feature
   <223> Synthetic DNA.
<400> 110
   tcccattaac tttcccaaac ct 22
<210> 111
   <211> 22
   <212> DNA
   <213> Artificial
<220>
   <221> misc_feature
   <223> Synthetic DNA.
<400> 111
   tcatcttcac caacccctca tt 22
<210> 112
   <211> 22
   <212> DNA
   <213> Artificial
<220>
   <221> misc_feature
   <223> Synthetic DNA.
<400> 112
   tctacacaaa acatttccct ac 22
<210> 113
   <211> 22
   <212> DNA
   <213> Artificial
<220>
   <221> misc_feature
   <223> Synthetic DNA.
<400> 113
   catcttacat aatatcttct at 22
<210> 114
   <211> 22
   <212> DNA
   <213> Artificial
<220>
   <221> misc_feature
   <223> Synthetic DNA.
<400> 114
   atccatccca ttcgactttc cc 22
<210> 115
   <211> 22
   <212> DNA
   <213> Artificial
<220>
   <221> misc_feature
   <223> Synthetic DNA.
<400> 115
   tacaacaatt ttctaaccat aa 22
<210> 116
   <211> 22
   <212> DNA
   <213> Artificial
<220>
   <221> misc_feature
   <223> Synthetic DNA.
<400> 116
   ccctcacact atcataccta ct 22
<210> 117
   <211> 22
   <212> DNA
   <213> Artificial
<220>
   <221> misc_feature
   <223> Synthetic DNA.
<400> 117
   tctcaattac tacatttcac ca 22
<210> 118
   <211> 22
   <212> DNA
   <213> Artificial
<220>
   <221> misc_feature
   <223> Synthetic DNA.
<400> 118
   acttctttta cctctcttct tt 22
<210> 119
   <211> 22
   <212> DNA
   <213> Artificial
<220>
   <221> misc_feature
   <223> Synthetic DNA.
<400> 119
   atttctttcc tttatcattt ta 22
<210> 120
   <211> 22
   <212> DNA
   <213> Artificial
<220>
   <221> misc_feature
   <223> Synthetic DNA.
<400> 120
   aattactttt tcttttccat ta 22
<210> 121
   <211> 22
   <212> DNA
   <213> Artificial
<220>
   <221> misc_feature
   <223> Synthetic DNA.
<400> 121
   accttattta cactaaacat tt 22
<210> 122
   <211> 22
   <212> DNA
   <213> Artificial
<220>
   <221> misc_feature
   <223> Synthetic DNA.
<400> 122
   atacaacttt caacttccta tt 22
<210> 123
   <211> 22
   <212> DNA
   <213> Artificial
<220>
   <221> misc_feature
   <223> Synthetic DNA.
<400> 123
   tctattcttt tcactccaat cc 22
<210> 124
   <211> 22
   <212> DNA
   <213> Artificial
<220>
   <221> misc_feature
   <223> Synthetic DNA.
<400> 124
   ttacaccttc actaaatcac ta 22
<210> 125
   <211> 22
   <212> DNA
   <213> Artificial
<220>
   <221> misc_feature
   <223> Synthetic DNA.
<400> 125
   tctattttaa tctctaacct tt 22
<210> 126
   <211> 22
   <212> DNA
   <213> Artificial
<220>
   <221> misc_feature
   <223> Synthetic DNA.
<400> 126
   ttttccacac actcctttcc at 22
<210> 127
   <211> 22
   <212> DNA
   <213> Artificial
<220>
   <221> misc_feature
   <223> Synthetic DNA.
<400> 127
   ttattttatt cttctaatcc tc 22
<210> 128
   <211> 22
   <212> DNA
   <213> Artificial
<220>
   <221> misc_feature
   <223> Synthetic DNA.
<400> 128
   ttcttcaaac acacacatta tt 22
<210> 129
   <211 > 22
   <212> DNA
   <213> Artificial
<220>
   <221> misc_feature
   <223> Synthetic DNA.
<400> 129
   cctctttatt aatatcttct ct 22
<210> 130
   <211> 22
   <212> DNA
   <213> Artificial
<220>
   <221> misc_feature
   <223> Synthetic DNA.
<400> 130
   tactcattta tctccttttc ta 22
<210> 131
   <211> 22
   <212> DNA
   <213> Artificial
<220>
   <221> misc_feature
   <223> Synthetic DNA.
<400> 131
   caccatcaat ccactatatt tc 22
<210> 132
   <211> 22
   <212> DNA
   <213> Artificial
<220>
   <221> misc_feature
   <223> Synthetic DNA.
<400> 132
   taattattct acttcaattt tt 22
<210> 133
   <211> 22
   <212> DNA
   <213> Artificial
<220>
   <221> misc_feature
   <223> Synthetic DNA.
<400> 133
   atcatctact cacaacccct ta 22
<210> 134
   <211> 22
   <212> DNA
   <213> Artificial
<220>
   <221> misc_feature
   <223> Synthetic DNA.
<400> 134
   tcttcttatt actatacttc ct 22
<210> 135
   <211> 22
   <212> DNA
   <213> Artificial
<220>
   <221> misc_feature
   <223> Synthetic DNA.
<400> 135
   atcacttaaa ccttctcact ta 22
<210> 136
<211> 90
<212> DNA
<213> Artificial
<220>
<221> misc_feature
   <223> Synthetic DNA.
<400> 136
   ctcacctatc tccccaacac ctaataacat tcaatcactc tttccactaa ccacctatct 60
   acatcaccaa atggaagacg ccaaaaacat 90
<210> 137
<211> 90
<212> DNA
<213> Artificial
<220>
<221> misc_feature
<223> Synthetic DNA.
<400> 137
   ctcacctatc tcctataaac ccaccttacc aatctccaca ttcaatatct ctccccttac 60
   cctcatcaca atggaagacg ccaaaaacat 90
<210> 138
<211> 90
<212> DNA
<213> Artificial
<220>
<221> misc_feature
   <223> Synthetic DNA.
<400> 138
   ctcacctatc tcccaacacc aataccaact ccactcacct atctccacct cacacacact 60
   tttccatcca atggaagacg ccaaaaacat 90
<210> 139
<211> 54
<212> DNA
<213> Artificial
<220>
<221> misc_feature
   <223> Synthetic DNA.
<400> 139
   ctcacctatc tccttatcct attttcctct tacaatggaa gacgccaaaa acat 54
<210> 140
<211> 54
<212> DNA
<213> Artificial
<220>
<221> misc_feature
   <223> Synthetic DNA.
<400> 140
   ctcacctatc tctcctatcc aaccatacat ccttatggaa gacgccaaaa acat 54
<210> 141
<211> 54
<212> DNA
<213> Artificial
<220>
<221> misc_feature
   <223> Synthetic DNA.
<400> 141
   ctcacctatc tctcccacat tcccccctat ctctatggaa gacgccaaaa acat 54
<210> 142
<211> 54
<212> DNA
<213> Artificial
<220>
<221> misc_feature
   <223> Synthetic DNA.
<400> 142
   ctcacctatc tcattttttc ttaattccct cattatggaa gacgccaaaa acat 54
<210> 143
<211> 54
<212> DNA
<213> Artificial
<220>
<221> misc_feature
   <223> Synthetic DNA.
<400> 143
   ctcacctatc tctctccact tttcttttac acccatggaa gacgccaaaa acat 54
<210> 144
<211> 54
<212> DNA
<213> Artificial
<220>
<221> misc_feature
   <223> Synthetic DNA.
<400> 144
   ctcacctatc tcacactatc aatacctact ctttatggaa gacgccaaaa acat 54
<210> 145
<211> 33
<212> DNA
<213> Artificial Sequence
<220>
<221> misc_feature
   <223> Synthetic DNA.
<400> 145
   cgatttaggt gacactatag aactcaccat ctc 33